# EUROPEAN PATENT SPECIFICATION

(11) **EP 2 065 629 B1**
(45) Date of publication and mention of the grant of the patent: **11.03.2015**
(21) Application number: 07806837.6
(22) Date of filing: 06.09.2007
(51) Int. Cl.: F16L 27/08, A61M 16/08, F16L 27/00, A61M 16/04

(54) **ROTARY CONNECTOR**
DREHVERBINDER
RACCORD TOURNANT

(43) Date of publication of application: 03.06.2009
(73) Proprietor: Senko Medical Instrument Mfg. Co., Ltd., Tokyo 113-0033 (JP)
(72) Inventor: UEHARA, Hirokazu, c/o SENKO MEDICAL INSTRUMENT Mfg. Co. Ltd., Tokyo 113-0033 (JP); TUKAKOSI, Syouichi, c/o SENKO MEDICAL INSTRUMENT Mfg. Co. Ltd., Tokyo 113-0033 (JP); YOKOI, Hiroshi, c/o SENKO MEDICAL INSTRUMENT Mfg. Co. Ltd., Tokyo 113-0033 (JP)
(74) Representative: Potter Clarkson LLP
(86) International application number: PCT/JP2007/067396
(87) International publication number: WO 2009/031223

(56) References cited:
- EP-A1- 1 479 405
- JP-A- 2007 182 925
- JP-U- 47 015 209
- JP-U- 61 148 342
- US-A- 4 033 353

## Description

### TECHNICAL FIELD

The present invention relates to a rotating connector for medical use to connect an external circuit with liquid medication tube, blood tube, or tracheal tube that is inserted into a human body by means of conical coupling.

Priority is claimed on Japanese Patent Application No. 2006-61889, filed on March 7, 2006,

### BACKGROUND ART

In many cases, connectors for medical use are required to have contradictory functions of providing secure coupling and providing easy attachability/detachability when coupling tube lines to each other or a tube line to a member. One means for satisfying this requirement is conical coupling. Many types of this conical coupling are specified in various standards of JIS and ISO which must be applied to medical products such as a circuit for artificial respiration, parts for a circuit, and liquid medication syringe.

Another requirement of connectors for medical use is that connection members be rotatable relative to each other while the above coupling is secured. It is rotating connectors for medical use that satisfy this requirement. Free rotation of their coupling members enables these connectors to avoid accidental clogging or unclogging of a tube line, or to avoid causing a pain for a patient.

Rotating connectors with various conical couplings which satisfy these requirements are now in use. However, conventional rotating connectors have a problem that it is difficult for them to be disconnected because of their rotatability.

For example, in conical coupling between a tracheal tube and an external circuit, a male terminal portion is provided that is mounted at an end portion of the tracheal tube and a female terminal portion is provided that is mounted at an end portion of an artificial respirator circuit, and these terminal portions are connected into an internationally standardized conical coupling state.

In this case, a rotating connector called a swivel connector is used in which a rotation mechanism is adopted so that stress due to twisting a respirator circuit or the like may be reduced for a patient who has a tracheal tube inserted in their throat.

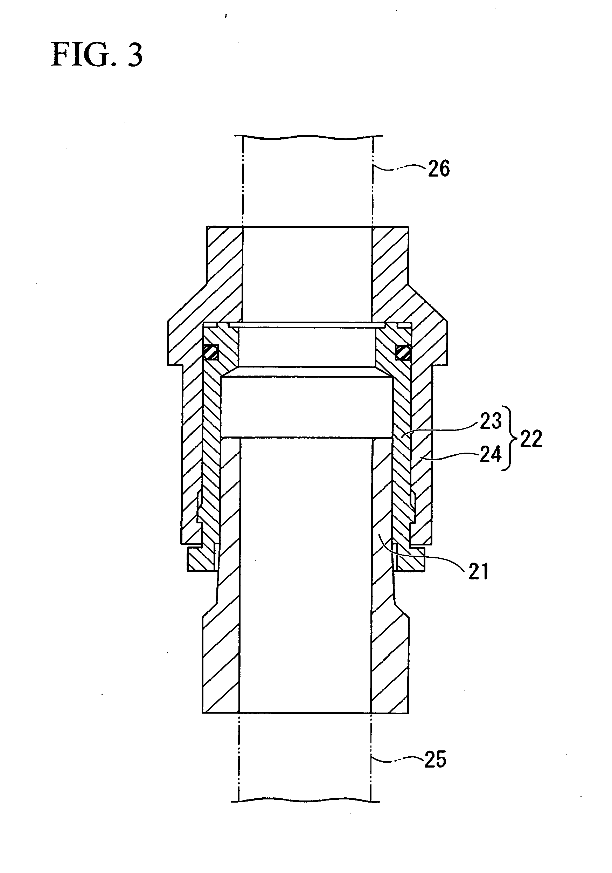

FIG. 3 shows a longitudinal cross-sectional view of a conventional rotating connector. The rotating connector is made of a male terminal portion 21 and a female terminal portion 22. The female terminal portion 22 is made of an internal barrel 23 that is connected to the male terminal portion 21, and an external barrel 24. The internal barrel 23 and external barrel 24 are made to be rotatable relative to each other. The male terminal portion 21 is mounted on a tracheal tube 25, and the female terminal portion 22 is mounted on an artificial respirator circuit 26. Thereby, the male terminal portion 21 is connected into the internal barrel 23 of the female terminal portion 22 in a conically coupled state.

It is necessary for these male terminal portion 21 and female terminal portion 22 to be disconnected extremely swiftly. However, as for conventional rotating connectors, even if the external barrel 24 is gripped and twisted by the fingers, it is difficult to disconnect the male terminal portion 21 from the female terminal portion 22 because the external barrel 24 rotates with respect to the internal barrel 23.

Therefore, as a connector allowing for easy disconnection with a rotation mechanism inside the connector, a single motion connector is proposed that adopts a latch mechanism (see Patent Document 1). A connector of this type is continuously used above the mouth or throat of a patient, and hence is preferably small and light weight. However, according to the single motion connector which adopts a latch mechanism described in Patent Document 1, a connector itself comes to have increased size and weight.

Patent Document 1: Japanese Unexamined Patent Application, First Publication No. 2004-344281

US 4 033 353 A provides a rotating connector comprising: a male terminal portion with a cone-shaped outer circumferential surface; and a female terminal portion with a cone-shaped inner circumferential surface that is connected with the cone-shaped outer circumferential surface in a conically coupled manner, wherein the female terminal portion includes: an internal barrel; and external barrel into which the internal barrel is rotatably inserted relative to the external barrel.

### DISCLOSURE OF THE INVENTION

### PROBLEMS SOLVED BY THE INVENTION

The present invention has an object to provide a connector which is capable of being conically coupled and easily disconnected while having a rotation mechanism inside the connector, and which is small in size and light in weight.

### MEANS FOR SOLVING THE PROBLEMS

To solve the above-mentioned problem, the present invention provides the following solutions.

A rotating connector according to the present invention includes a male terminal portion with a cone-shaped outer circumferential surface, and a female terminal portion with a cone-shaped inner circumferential surface that is connected with the cone-shaped outer circumferential surface in a conically coupled manner, in which either one of the female terminal portion or the male terminal portion includes an internal barrel, and an elastically deformable external barrel into which the internal barrel is rotatably inserted relative to the external barrel, and an engaging device is provided between the outer circumferential surface of the internal barrel and the inner circumferential surface of the external barrel, the engaging device coming in contact with the circumferential surfaces by elastic deformation of the external barrel to enable restraint of the relative rotation.

Moreover, in the rotating connector according to the present invention, the engaging device is made of protrusions formed on either one of the outer circumferential surface of the internal barrel and the inner circumferential surface of the external barrel; and recess portions are formed in the other, the recess portions being capable of engaging with the protrusions.

Either one of the male terminal portion and the female terminal portion is constructed as a dual barrel made of an internal barrel and an external barrel that is rotatable relative thereto, which provides the rotating connector with a rotation mechanism. Furthermore, it is possible to restrain the relative rotation between the internal barrel and the external barrel by gripping the external barrel with the fingers making it elastically deform. Therefore, it is possible to easily disconnect the male terminal portion from the conically coupled female terminal portion by pulling the external barrel while gripping and twisting it.

In the rotating connector according to the present invention, it is preferable that the female terminal portion be provided with the internal barrel having the cone-shaped internal circumferential surface and the elastically deformable external barrel into which the internal barrel is rotatably inserted relative to the external barrel.

With a construction of the female terminal portion as a dual barrel made of an internal barrel with a cone-shaped inner circumferential surface and an external barrel that is rotatable relative thereto, it is possible to restrain the relative rotation between the internal barrel and the external barrel by gripping the external barrel, which is positioned most outside in the connector, with the fingers making it elastically deform.

When the external barrel is gripped making it elastically deform bringing the outer circumferential surface of the internal barrel into contact with the inner circumferential surface of the external barrel, the protrusions and the recess portions are engaged, enabling restraint of the relative rotation.

Furthermore, in the rotating connector according to the present invention, it is preferable that the protrusions be protrusion strips formed along an axis direction of the female terminal portion, and that the recess portions be grooves that can be engaged with these protrusion strips.

When the protrusions and the recess portions are formed as a combination of protrusion strips and grooves along the axis direction, it is possible to maintain the length in the axis direction. Therefore, it is possible to make the grip position wide which allows restraint of rotation when being gripped.

Moreover, it is preferable that the rotating connector according to the present invention be one for connecting a tracheal tube and an artificial respirator circuit.

When the rotating connector like this is used for connecting a tracheal tube and an artificial respirator circuit, it is possible to provide a connector which is capable of being conically coupled and easily disconnected while having a rotation mechanism inside the connector, and, which is small in size and light in weight.

### ADVANTAGEOUS EFFECT OF THE INVENTION

According to the present invention, it is possible to provide a connector which is capable of being conically coupled and easily disconnected, while having a rotation mechanism inside the connector, by pulling the external barrel of the female terminal portion while gripping and twisting it with the fingers, and, which is small in size and light in weight.

### BRIEF DESCRIPTION OF THE DRAWINGS

FIG 1 is a longitudinal cross-sectional view showing a rotating connector for a tracheal tube according to one embodiment of the present invention.
FIG 2A is a transverse cross-sectional view of the rotating connector for a tracheal tube of FIG. 1, taken along the line A-A'.
FIG 2B is a transverse cross-sectional view of the rotating connector for a tracheal tube of FIG 1, taken along the line A-A', when the external barrel of the female terminal portion is gripped by the fingers.
FIG 3 is a longitudinal cross-sectional view showing a conventional rotating connector.

### DESCRIPTION OF THE REFERENCE SYMBOLS

1: rotating connector for a tracheal tube (rotating connector)
2: male terminal portion
3: female terminal portion
4: cone-shaped outer circumferential surface
5: internal barrel
6: external barrel
7: cone-shaped inner circumferential surface
8: annular recess portion
9: annular protrusion portion
10: axis
11: protrusion strip
12: groove
13: groove formation region
14: protrusion strip formation region
15: tongue-shaped protrusion piece
16: sealing portion
51: tracheal tube
52: artificial respirator circuit
a: thickness of the protrusion strip formation region of the internal barrel
b: height of the protrusion strip
c: thickness of the groove formation region of the external barrel
d: depth of the groove

### BEST MODE FOR CARRYING OUT THE INVENTION

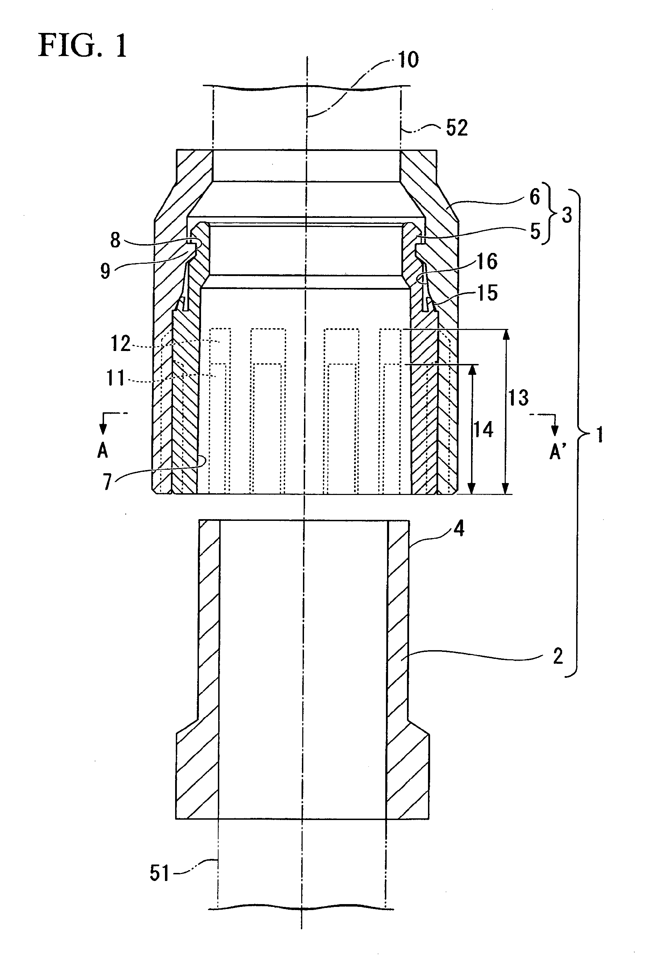

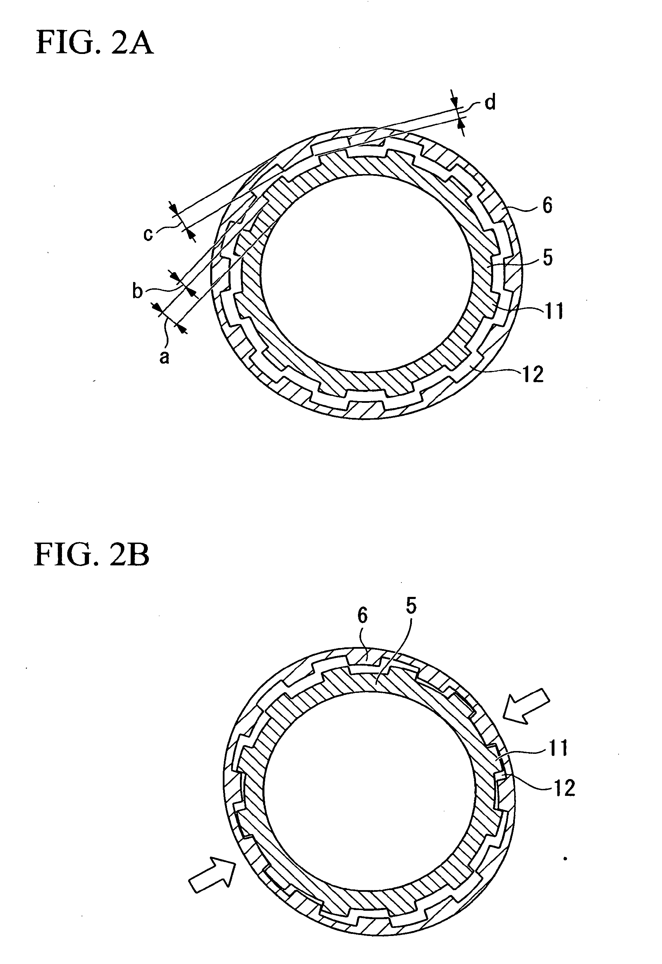

One embodiment of a rotating connector for a tracheal tube according to the present invention will be described based on FIG. 1, FIG. 2A, and FIG. 2B.

FIG. 1 shows a longitudinal cross-sectional view of the present embodiment. FIG. 2A shows a transverse cross-sectional view of FIG. 1, taken along the line A-A'. FIG. 2B shows a state in which an external barrel of a female terminal portion is gripped.

As shown in FIG. 1, a rotating connector for a tracheal tube 1 of the present embodiment is made of a male terminal portion 2 that is connected to an end of a tracheal tube 51, and a female terminal portion 3 that is connected to an end of an artificial respirator circuit 52. The male terminal portion 2 is generally formed into a barrel shape, and an outer circumstantial surface of a tip end portion except its base end portion is made as a cone-shaped outer circumferential surface 4 which tapers down slightly. On the other hand, the female terminal portion 3 is constructed as a dual barrel made of an internal barrel 5 and an external barrel 6 which are relatively rotatable. In the internal barrel 5, an internal circumferential surface except its base end portion is made as a cone-shaped inner circumferential surface 7 that is connected to the cone-shaped outer circumferential surface 4 of the male terminal portion 2 into a conically coupled state. Moreover, in the internal barrel 5 and the external barrel 6, on the side which is connected to the artificial respirator circuit 52, an annular recess portion 8 is formed in an outer circumferential surface of the internal barrel 5, and an annular protrusion portion 9 is formed in an inner circumferential surface of the external barrel 6. The annular recess portion 8 and annular protrusion portion 9 are brought into a fit state, to thereby prevent the internal barrel 5 from coming out of the external barrel 6.

As shown in FIG 2A, in the internal barrel 5 and the external barrel 6, at the tip end on the side for connection to the male terminal portion 2, a plurality of protrusion strips 11 which extend along an axis 10 direction of the internal barrel 5 are formed on the outer circumferential surface thereof over the whole circumference in a spaced-apart manner, and as many grooves 12 which are engageable with the respective protrusion strips 11 of the internal barrel 5 are formed on the inner circumferential surface of the external barrel 6 over the whole circumference in a spaced-apart manner for allowing engagement with the protrusion strips 11. In this case, the groove 12 is formed slightly larger than the protrusion strip 11.

Furthermore, a groove formation region 13 of the external barrel 6 is formed thin, allowing elastic deformation when being gripped. In this case, the length of the groove formation region 13 is formed longer than the width of a human finger.

Between the protrusion strip formation region 14 on the outer circumferential surface and the annular recess portion 8 of the internal barrel 5, a tongue-shaped protrusion piece 15 is formed along the circumferential direction. This tongue-shaped protrusion piece 15 is in elastic contact with a sealing portion 16 that is formed on an inner circumferential surface between the groove formation region 13 and the annular protrusion portion 9 of the external barrel 6. Thereby, air flowing inside the rotating connector for a tracheal tube 1 is prevented from leaking to the outside.

In a condition that the male terminal portion 2 and the female terminal portion 3 of the rotating connector for a tracheal tube 1 are connected, the male terminal portion 2 and the internal barrel 5 of the female terminal portion 3 are connected into a conically coupled state so as not to be easily disconnected. To release the connecting between the male terminal portion 2 and the female terminal portion 3, the female terminal portion 3 is moved in the artificial respirator circuit 52 direction while the external barrel 6 is gripped and twisted by the fingers, to thereby pull out the male terminal portion 2.

That is, when the groove formation region 13 of the external barrel 6 is gripped in the directions indicated by the white arrows as shown in FIG. 2B, the gripped portions are deflected together with their neighboring areas, to thereby bring these portions into contact with the outer circumferential surface of the internal barrel 5. Then, the protrusion strips 11 formed on the outer circumferential surface of the internal barrel 5 are engaged with the grooves 12 formed in the inner circumferential surface of the external barrel 6 to restrain the relative rotation between the internal barrel 5 and the external barrel 6.

With twisting force applied to the external barrel 6, it is possible to rotate the internal barrel 5 together with the external barrel 6 and to easily disconnect the internal barrel 5 from the male terminal portion 2. That is, the protrusion strips 11 and the grooves 12 constitute engaging device of the present invention.

Even if the protrusion strips are not fit into the grooves 12 when the groove formation region 13 of the external barrel 6 is gripped, the external barrel 6 is rotatable with respect to the internal barrel 5. Therefore, when a twist is applied, the grooves formed in the inner circumferential surface of the external barrel 6 is moved on the outer circumferential surface of the internal barrel 5 along the circumference, and the protrusion strips 11 formed on the outer circumferential surface of the internal barrel 5, and eventually fit into the grooves 12 to restrain the relative rotation between the internal barrel 5 and the external barrel 6.

In the small and light rotating connector for a tracheal tube that is capable of being conically coupled, while having a rotation mechanism inside the connector, it is possible to disconnect the female terminal portion from the male terminal portion by pulling the female terminal portion while gripping and twisting it with the fingers.

In the case where the rotating connector for a tracheal tube of the present embodiment is applied to a product under an international standard for 22 mm/15 mm coaxial conical coupling, for example polypropylene resin with a Rockwell hardness factor of 80 to 105 is used for the male terminal portion 2 and the female terminal portion 3. Moreover, the internal barrel 5 has an inner diameter of about 15 mm, and the external barrel 6 has an outer diameter of about 22 mm. In the protrusion strip formation region 14, the internal barrel 5 has a thickness a of 1.4 mm and the protrusion strip has a height b of 0.6 mm, while in the groove formation region 13, the external barrel 6 is thinly formed with a thickness c of 1.0 mm so as to facilitate elastic deformation and the groove 12 has a depth d of 0.5 mm.

As a material for this rotating connector, any crystalline resin, in addition to polypropylene resin, may be used as long as it does not break when it is deformed. Different materials may be combined as necessary, such as a hard material for the internal barrel 5 and a soft material for the external barrel 6.

It is possible to use the rotating connector according to the present invention not only for a tracheal tube, but also for a tube for tracheotomy. Therefore, a tracheal tube in the present invention includes a tracheotomy tube.

The rotating connector according to the present invention may be used not only for connection of an outside circuit to a tube through which a gas flows such as the rotating connector for a tracheal tube of the present embodiment, but also in a transfusion line of a liquid such as liquid medication or blood.

The engaging device according to the present invention is made of the protrusion strips 11 and the grooves 12 that extend along the axis direction of the internal barrel 5 and the external barrel 6. However, the engaging device does not need to be one that is formed along the axis direction as long as it can restrain relative rotation between the barrels as a result of their engagement when the external barrel is gripped. For example, a combination of simple protrusions and recess portions, or coarse surfaces with fine asperities, or the like may be used.

Furthermore, in this embodiment of the rotating connector according to the present invention, a rotation mechanism and a restraining device thereof are provided in the female terminal portion. However, these may be provided in the male terminal portion.

In this embodiment of the rotating connector according to the present invention, the rotating connector is applied to 22 mm/15 mm coaxial conical coupling. However, the application is not limited thereto. This rotating connector may be applied to such a 15 mm conical connector, a 22 mm conical connector, a 30 mm conical connector, or a conical connector for a medical device including a Luer connector, an elbow connector, and an extension connector, as is adopted in a circuit for artificial respiration, in a part for a circuit such as for an artificial nose (heat and moisture exchanger: HME), a water trap, or a filter, and in a product for medical use such as for liquid medication syringe, an enteral nutrition line, or a flow sensor.

### INDUSTRIAL APPLICABILITY

As described above, according to the present invention, it is possible to provide a connector which is capable of being conically coupled and easily disconnected, while having a rotation mechanism inside the connector, by pulling the external barrel of the female terminal portion while gripping and twisting the external barrel with the fingers, and, which is small in size and light in weight.

## Claims

1. A rotating connector (1) comprising:
a male terminal portion (2) with a cone-shaped outer circumferential surface; and a female terminal portion (3) with a cone-shaped inner circumferential surface that is connected with the cone-shaped outer circumferential surface in a conically coupled manner, wherein
either one of the female terminal portion (2) or the male terminal portion (3) includes: an internal barrel; and an external barrel into which the internal barrel is rotatably inserted relative to the external barrel, and
**characterized in that**,
the external barrel is elastically deformable,
an engaging device (11, 12) is provided between the outer circumferential surface of the internal barrel and the inner circumferential surface of the external barrel, the engaging device coming in contact with the circumferential surfaces by elastic deformation of the external barrel to enable restraint of the relative rotation, and
the engaging device (11, 12) is made of: protrusions (11) formed on either one of the outer circumferential surface of the internal barrel (5) and the inner circumferential surface of the external barrel (6); and recess portions (12) formed in the other, the recess portions (12) being capable of engaging with the protrusions (11).

2. The rotating connector (1) according to claim 1, wherein the female terminal portion (3) is provided with the internal barrel (5) having the cone-shaped internal circumferential surface and the elastically deformable external barrel (6) into which the internal barrel (5) is rotatably inserted relative to the external barrel (6).

3. The rotating connector (1) according to claim 1, wherein the protrusions (11) are protrusion strips formed along an axis direction of the female terminal portion, and the recess portions (12) are grooves that can be engaged with these protrusion strips.

4. The rotating connector (1) according to any one of claim 1 to claim 3, being one for connecting a tracheal tube (51) and an artificial respirator circuit (52).

## Patentansprüche

1. Ein Drehverbinder (1) mit:
einem männlichen Anschlussteil (2) mit einer konusförmigen äußeren Umfangsfläche; und einem weiblichen Anschlussteil (3) mit einer konusförmigen inneren Umfangsfläche, die mit der konusförmigen äußeren Umfangsfläche in einer konisch gekoppelten Weise verbunden ist, wobei einer von dem weiblichen Anschlussteil (2) und dem männlichen Anschlussteil (3) ein Innenrohr und ein Außenrohr umfasst, in das das Innenrohr relativ zu dem Außenrohr drehbar eingesetzt ist, und **dadurch gekennzeichnet, dass**
das Außenrohr elastisch deformierbar ist,
eine Angriffsvorrichtung (11, 12) zwischen der äußeren Umfangsfläche des Innenrohrs und der inneren Umfangsfläche des Außenrohrs vorgesehen ist, wobei die Angriffsvorrichtung durch elastische Deformation des Außenrohrs in Kontakt mit den Umfangsflächen kommt, um eine Einschränkung der Relativverdrehung zu ermöglichen, und
die Angriffsvorrichtung (11, 12) aus Vorsprüngen (11), die auf einer der äußeren Umfangsfläche des Innenrohrs (5) und der inneren Umfangsfläche des Außenrohrs (6) ausgeformt sind. und rückspringenden Bereichen (12) ausgebildet ist, die in der anderen Umfangsfläche ausgebildet sind, wobei die rückspringenden Bereiche (12) in der Lage sind, mit den Vorsprüngen (11) in Eingriff zu gelangen.

2. Der Drehverbinder (1) nach Anspruch 1, wobei der weibliche Anschlussteil (3) mit dem Innenrohr (5), das die konusförmige innere Umfangsfläche aufweist, und dem elastisch deformierbaren Außenrohr (6) versehen ist, in das das Innenrohr (5) relativ zu dem Außenrohr (6) verdrehbar eingesetzt ist.

3. Der Drehverbinder (1) nach Anspruch 1, wobei die Vorsprünge (11) Vorsprungsstreifen sind, die längs einer Achsenrichtung des weiblichen Anschlussteils ausgebildet sind, und die rückspringenden Bereiche (12) Nuten sind, die mit den Vorsprungsstreifen in Eingriff gebracht werden können.

4. Der Drehverbinder (1) nach einem der Ansprüche 1 bis 3, der ein solcher zum Verbinden eines Trachealrohrs (51) und eines Kreislaufs (52) eines Geräts zur künstlichen Beatmung ist.

## Revendications

1. Connecteur rotatif (1) comprenant :
une partie de borne mâle (2) avec une surface circonférentielle externe de forme conique ; et une partie de borne femelle (3) avec une surface circonférentielle interne de forme conique qui est raccordée avec la surface circonférentielle externe de forme conique d'une manière coniquement couplée, dans lequel
l'une parmi la partie de borne femelle (2) ou la partie de borne mâle (3) comprend :
un corps cylindrique interne ; et un corps cylindrique externe dans lequel le corps cylindrique interne est inséré en rotation par rapport au corps cylindrique externe, et
**caractérisé en ce que**
le corps cylindrique externe est élastiquement déformable,
un dispositif de mise en prise (11, 12) est prévu entre la surface circonférentielle externe du corps cylindrique interne et la surface circonférentielle interne du corps cylindrique externe, le dispositif de mise en prise venant en contact avec les surfaces circonférentielles par déformation élastique du corps cylindrique externe pour permettre la retenue de la rotation relative, et
le dispositif de mise en prise (11, 12) est réalisé à partir de : saillies (11) formées sur l'une parmi la surface circonférentielle externe du corps cylindrique interne (5) et la surface circonférentielle interne du corps cylindrique externe (6) ; et des parties d'évidement (12) formées dans l'autre, les parties d'évidement (12) pouvant se mettre en prise avec les saillies (11).

2. Connecteur rotatif (1) selon la revendication 1, dans lequel la partie de borne femelle (3) est prévue avec le corps cylindrique interne (5) ayant la surface circonférentielle interne de forme conique et le corps cylindrique externe élastiquement déformable (6) dans lequel le corps cylindrique interne (5) est inséré en rotation par rapport au corps cylindrique externe (6).

3. Connecteur rotatif (1) selon la revendication 1, dans lequel les saillies (11) sont des bandes de saillie formées le long d'une direction axiale de la partie de borne femelle et les parties d'évidement (12) sont des rainures qui peuvent être mises en prise avec ces bandes de saillie.

4. Connecteur rotatif (1) selon l'une quelconque des revendications 1 à 3, qui est un connecteur pour raccorder un tube trachéal (51) à un circuit de respirateur artificiel (52).
